(19) Europäisches Patentamt
European Patent Office
Office européen des brevets

(11) Veröffentlichungsnummer : **0 024 648**
**B1**

(12) **EUROPÄISCHE PATENTSCHRIFT**

(45) Veröffentlichungstag der Patentschrift : **26.01.83**

(21) Anmeldenummer : **80104827.3**

(22) Anmeldetag : **14.08.80**

(51) Int. Cl.³ : **C 07 C 29/136**, C 07 C 29/14, C 07 C 33/02, C 07 C 33/025, C 07 C 33/03, B 01 J 23/40

# (54) Verfahren zur Herstellung von ungesättigten Alkoholen.

(30) Priorität : **24.08.79 DE 2934251**

(43) Veröffentlichungstag der Anmeldung : **11.03.81 Patentblatt 81/10**

(45) Bekanntmachung des Hinweises auf die Patenterteilung : **26.01.83 Patentblatt 83/04**

(84) Benannte Vertragsstaaten : **BE CH DE FR GB IT LI NL**

(56) Entgegenhaltungen :
**DE A 2 357 645**
**FR A 1 489 504**
**US A 3 655 777**

**CHEMICAL ABSTRACTS, Band 87, Nr. 19, 7. November 1977, Seite 554, Nr. 151800h Columbus, Ohio, U.S.A. T. MIZOROKI et al. : « Rhodium complex catalyzed hydrogenation of alpha-beta-unsaturated aldehydes to unsaturated alcohols ».**
**CHEMICAL ABSTRACTS, Band 87, Nr. 25, 19. Dezember 1977, Seite 664, Nr. 200774p Columbus, Ohio, U.S.A.**
**CHEMICAL ABSTRACTS, Band 89, Nr. 23, 4. Dezember 1978, Seite 582, Nr. 197020y Columbus, Ohio, U.S.A. G. MESTRONI et al. : « Selective hydrogenation of unsaturated ketones by rhodium (I) complexes containing a 2,2'-bipyridine ligand ».**
**CHEMICAL ABSTRACTS, Band 90, Nr. 19. 7. Mai 1979, Seite 633, Nr. 152369y Columbus, Ohio, U.S.A. D. V. SOKOL'SKII et al. : « Hydrogenation of citral to citronellol on Group VIII metals ».**

(73) Patentinhaber : **BASF Aktiengesellschaft**
**Carl-Bosch-Strasse 38**
**D-6700 Ludwigshafen (DE)**

(72) Erfinder : **Horner, Michael, Dr.**
**Pfalzgrafenstrasse 15B**
**D-6730 Neustadt (DE)**
Erfinder : **Irgang, Matthias, Dr.**
**Gontardstrasse 4**
**D-6800 Mannheim 1 (DE)**
Erfinder : **Nissen, Axel, Dr.**
**Panoramastrasse 51**
**D-6906 Leimen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 024 648 B1

Jouve, 18, rue St-Denis, 75001 Paris, France

# Verfahren zur Herstellung von ungesättigten Alkoholen

Die vorliegende Erfindung betrifft ein verbessertes Verfahren zur Herstellung von olefinisch ungesättigten Alkoholen (I) durch selektive Hydrierung der entsprechenden Carbonylverbindungen (II) mit Wasserstoff in Gegenwart eines Ruthenium-, Rhodium-, Osmium-, Iridium- oder Platin-Katalysators in der Flüssigphase.

Speziell betrifft die Erfindung die Herstellung von ungesättigten Alkoholen der allgemeinen Formel Ia

$$R^1-\overset{R^2}{\overset{|}{C}}=\overset{R^3}{\overset{|}{C}}-\overset{R^4}{\overset{|}{C}}H-OH \qquad (Ia)$$

in der $R^1$ Wasserstoff oder einen organischen Rest und $R^2$, $R^3$ und $R^4$ Wasserstoff oder eine $C_1$-$C_4$-Alkylgruppe bedeuten, durch selektive Hydrierung von α,β-ungesättigten Carbonylverbindungen (IIa)

$$R^1-\overset{R^2}{\overset{|}{C}}=\overset{R^3}{\overset{|}{C}}-\overset{R^4}{\overset{|}{C}}=O \qquad (IIa)$$

wobei der Hydrierung von Citral (IIb)

$$CH_3-\overset{CH_3}{\overset{|}{C}}=CH-CH_2-CH_2-\overset{CH_3}{\overset{|}{C}}=CH-CHO \qquad (IIb)$$

zu Geraniol und Nerol (E bzw. Z-3,7-Dimethylocta-2,6-dien-1-ol ; E-Ib und Z-Ib)

$$CH_3-\overset{CH_3}{\overset{|}{C}}=CH-CH_2-CH_2-\overset{CH_3}{\overset{|}{C}}=CH-CH_2OH \qquad (Ib)$$

besondere Bedeutung zukommt.

Aus der Arbeit von Bakhanova et al, Izv. Akad. Nauk SSR, Ser Khim 9 (1972), S. 1934f, ist es bekannt, Geraniol (E-Ib) durch Hydrierung von Citral IIb mit Wasserstoff in Gegenwart eines Iridiumkatalysators herzustellen. Dieses Verfahren hat jedoch den Nachteil, daß man hierzu unverhältnismässig viel des teuren Edelmetalls benötigt, nämlich etwa 4 Gew.% Iridium, bezogen auf den zu hydrierenden Aldehyd. Ebenso unvorteilhaft ist Osmium als Katalysator für die Hydrierung ungesättigter Aldehyde, wie sie in Tetrahedron Letters Nr. 20, 1969, Seite 1579f empfohlen wird. Hier benötigt man 1 g eines Os/Aktivkohlekatalysators mit 5 Gew.% Os zur Hydrierung von 10 g Zimtaldehyd, d.h. die Menge an reinem Osmium beträgt 0,5 Gew.%, ein Wert, der für technisch-wirtschaftliche Zwecke viel zu hoch ist.

Weiterhin ist es aus J. Am. Chem. Soc. 47 (1925), S. 3061f, bekannt, Zimtaldehyd mittels Platinkatalysatoren zu hydrieren, jedoch ist hierbei die Selektivität bezüglich des Zimtalkohols unbefriedigend. Nur wenn zur Hydrierung ungesättigter Aldehyde neben Platin noch andere Katalysatorkomponenten wie Eisen und Zink (BE-PS 837 057), Eisen und Silber (US-PS 3 284 517) oder Cobalt (DE-OS 2 412 517) mitverwendet werden, läßt sich die Formylgruppe gezielt hydrieren. Derartige Katalysatorsysteme sind jedoch sehr empfindlich und verlieren entweder schnell ihre Aktivität oder ihre selektive Wirkung. Diese Beobachtung deckt sich mit der Feststellung in der DE-OS 2 650 046, Seite 11, nach welcher der Mangel dadurch behoben werden soll, daß man Platinkatalysatoren verwendet, die durch Vorbehandlung mit Wasserstoff bei höheren Temperaturen hergestellt worden sind. Diese Vorbehandlung ist für technische Zwecke jedoch umständlich, da sie entweder lange dauert oder einen hohen Wasserstoffdruck erfordert. Außerdem wird das Hydrierproblem hierdurch nicht befriedigend gelöst, denn setzt man einen derartigen Katalysator wiederholt ein, so erhält man von Zyklus zu Zyklus bei unterschiedlichen Reaktionszeiten stark unterschiedliche Ergebnisse so daß ein stetiger Betriebsablauf praktisch kaum möglich ist.

Ferner werden ungesättigte Aldehyde nach der allgemeinen Lehre der FR-PS 1 489 504 mittels Platinkatalysatoren in Gegenwart von Alkalihydroxiden oder -alkoholaten in alkoholischer Lösung zu den entsprechenden ungesättigten Alkoholen hydriert. Nachteilig bei diesem am Beispiel des Zimtaldehyds erläuterten Verfahrens ist jedoch, daß die hierbei nach Abtrennung des Katalysators anfallenden Reaktionsgemische wegen der zunächst noch in Lösung befindlichen salzartigen Alkaliverbindungen großtechnisch nicht oder nur unter erheblichen Schwierigkeiten destillativ aufgearbeitet werden können und daß extraktive Methoden einen zusätzlichen Verfahrensaufwand bedingen. Trotz dieses evidenten Nachteils ist dieser FR-PS keine Anregung zu dem weiter unten definierten erfindungsgemäßen Verfahren zu entnehmen.

Nach dem Verfahren der US-PS 3 655 777 werden α,β-ungesättigte Aldehyde mit Hilfe von Osmium-Trägerkatalysatoren zu den entsprechenden ungesättigten Alkoholen hydriert. Befriedigend gelingt dieses Verfahren jedoch nur im Falle des Zimtaldehyds, wogegen die Selektivitäten im Falle von Crotonaldehyd und Acrolein erheblich zu wünschen übrig lassen. Das Verfahren dieser US-PS stellt daher keine allgemeine brauchbare Methode dar und ist daher verbesserungsbedürftig.

Gemäß Chemical Abstracts, Band 90, 1979, Seite 633, Referat 152 369y liefert die Hydrierung von Citral mit Rhodiumkatalysatoren hauptsächlich Citronellol (3,7-Dimethyloct-2-en-1-ol), mit Platinkatalysatoren offensichtlich ein sehr uneinheitliches Reaktionsgemisch und mit Palladiumkatalysatoren überwiegend den gesättigten Aldehyd. Als weitere Hydrierungsprodukte wurden Citronellal (3,7-Dimethyloct-2-en-1-al) und der entsprechende gesättigte Alkohol gefunden, nicht aber Geraniol bzw. Nerol. Dieser Arbeit zufolge ist die selektive katalytische Hydrierung von Citral zu Geraniol also nicht möglich.

Der Erfindung lag daher die Aufgabe zugrunde, bei der Hydrierung der ungesättigten Aldehyde II zu den entsprechenden ungesättigten Alkoholen I die Selektivität dieser Reaktion mit möglichst geringem technisch-wirtschaftlichem Aufwand zu erhöhen und sowohl Aktivität als auch Selektivität über lange Betriebszeiten mit ebenfalls geringem Aufwand zu gewährleisten.

Dementsprechend wurde ein verbessertes Verfahren zur Herstellung der ungesättigten Alkohole I durch selektive Hydrierung der Carbonylverbindungen II mit Wasserstoff in Gegenwart von Edelmetallkatalysatoren gefunden, welches dadurch gekennzeichnet ist, daß man

— als Katalysator einen Ruthenium-, Rhodium-, Osmium-, Iridium- oder Platin-Katalysator verwendet und daß man

— die Hydrierung in Gegenwart von 5 bis 40 Gew.%, bezogen auf (II), eines tertiären Amins vornimmt.

Die erfindungsgemäße Maßnahme gestattet bei verbessertem Verfahrenserfolg eine erhebliche Einsparung an den genannten Edelmetall-Katalysatoren, verglichen mit den herkömmlichen Arbeitsweisen. Außerdem wird die Selektivität bezüglich der Verfahrensprodukte I verbessert und über lange Betriebszeiten auf ihrem ursprünglich hohen Niveau gehalten, was vor allem für Duft- und Aromastoffe, z.B. das Citronellal, von Bedeutung ist, denn bei einer mit wirtschaftlich vertretbaren Aufwand vorgenommenen Reindarstellung des Produktes sinkt die Ausbeute an den gewünschten Verfahrensprodukten erheblich.

Prinzipiell sind nach den bisherigen Beobachtungen jegliche tertiäre Amine geeignet, so daß es auf deren chemische Natur, sofern sie aufgrund funktioneller Gruppen nicht anderweitig mit den Reaktionspartnern reagieren können, nicht ankommt. Genannt seien beispielsweise

— aliphatische tertiäre Amine mit insgesamt 3 bis 30 C-Atomen, wie vor allem Trimethylamin sowie Triethylamin, Triethanolamin und Trihexylamin,

— cyclische tertiäre Amine wie N-Methylpiperidin, N-Methylmorpholin und N,N'-Dimethylpiperazin

— aliphatisch-cycloaliphatische tertiäre Amine wie N,N-Dimethylcyclohexylamin

— aliphatisch-araliphatische tertiäre Amine wie N,N-Dimethylbenzylamin

— aliphatisch-aromatische tertiäre Amine wie N,N-Dimethylanilin sowie

— heterocyclisch-aromatische tertiäre Amine wie Pyridin und Chinolin.

Aus wirtschaftlichen Gründen wird man in übrigen möglichst billige Amine verwenden, deren Siedepunkt entweder deutlich tiefer oder deutlich höher ist, als der des Verfahrensproduktes, da sich in diesen Fällen entweder die Amine oder die Verfahrensprodukte leicht aus dem Reaktionsgemisch abdestillieren lassen.

Die Menge der Amine beträgt vorzugsweise 25 bis 40 Gew.% des Einsatzstoffes II.

Als Hydrierkatalysatoren eignen sich alle Edelmetallkatalysatoren der VIII. Gruppe des Periodensystems mit Ausnahme des Palladiums, durch welches vornehmlich die olefinische Gruppierung in α,β-Stellung hydriert wird. Die übrigen definitionsgemäß zu verwendenden Edelmetalle können als Substanz- oder vorzugsweise als Trägerkatalysatoren eingesetzt werden, wobei es ein besonderer Vorteil ist, daß sich nach den bisherigen Beobachtungen sämtliche handelsüblichen Katalysatoren gleichermaßen gut eignen. Die Betriebsbedingungen brauchen daher bei Wechsel der Katalysatorart entweder gar nicht oder nur geringfügig geändert zu werden. Hinsichtlich der Aktivität und der Selektivität besonders gute Eigenschaften werden mit Ruthenium/Aktivkohle-Katalysatoren erzielt, die eine innere Oberfläche (gemessen nach BET) von 600-900 $m^2/g$ haben.

Bezogen auf den Metallgehalt und auf die Menge der Ausgangsverbindungen II setzt man die Metalle vorzugsweise in Mengen von 0,000 1-0,1, besonders 0,001-0,1 Gew.% ein.

Da die meisten handelsüblichen Katalysatoren Trägerkatalysatoren mit Aktivkohle als Träger und 5 Gew.% Metallgehalt sind, werden derartige Katalysatoren aus wirtschaftlichen Gründen bevorzugt.

Es empfiehlt sich, die Reaktion in Gegenwart eines Lösungsmittels vorzunehmen. Die Mengen an Lösungsmittel liegen im allgemeinen zwischen 10 und 300, vorzugsweise 25 und 150 Gew.% von II. Als Lösungsmittel eignen sich alle inerten Flüssigkeiten, in denen sowohl I und II als auch das mitverwendete tertiäre Amin löslich sind. In Betracht kommen beispielsweise die tertiären Amine selber sowie zusätzlich Alkohole wie Methanol und Ethanol, Ether, Aceton und unter den Reaktionsbedingungen flüssige Kohlenwasserstoffe wie Hexan und Cyclohexan. Bevorzugt wird Methanol, und zwar vor allem, wenn man Trimethylamin als Base verwendet, da sich in diesem Falle die Aufarbeitung der Reaktionsgemische besonders einfach gestaltet.

Im übrigen nimmt man die Hydrierung wie üblich vor, also bei Temperaturen zwischen 20 und 150 °C und einem Druck von 20 bis 200 bar, wenn man weniger als 0,1 Gew.% Katalysatormetall (bezogen auf II) verwendet, und einem Druck von 1 bis 150 bar, wenn die Katalysatormenge größer ist als 0,1 Gew.%.

Besondere Bedeutung hat das erfindungsgemäße Verfahren für die bekanntermaßen technisch problematische Hydrierung von Citral IIb zu Geraniol bzw. Nerol (E-Ib bzw. Z-Ib), da hier Mehrfachhydrierung und Isomerisierung als Konkurrenzreaktionen auftreten.

Ebensogut ist das Verfahren aber auch auf die sonstigen Verbindungen II anwendbar, wobei es meistens auch die genannten Vorteile über die bisher bekannten Verfahren bietet, wie es fast, durchweg für die definitionsgemäßen Verbindungen IIa zutrifft, deren olefinische Doppelbindung wegen ihrer Konjugation zur Carbonylgruppe besonders leicht hydriert wird.

Die Reste $R^1$ in den Verbindungen IIa können prinzipiell beliebig sein. Enthalten diese Reste weitere olefinische Doppelbindungen, so werden diese nicht angegriffen. Beispiele für $R^1$ sind Alkyl- und Alkenylreste mit 1 bis 20 C-Atomen und aromatische Reste wie die Phenylgruppe. Diese Reste können ihrerseits beispielsweise Alkylgruppen, Alkoxygruppen, Carbalkoxygruppen, Acylgruppen, Hydroxylgruppen, Carboxylgruppen, Nitrilgruppen, Aminogruppen und Halogen als Substituenten tragen. Da das Verfahrensprinzip von der Art der Substituenten $R^1$ bis $R^3$ nicht berührt wird, erübrigt sich eine gesonderte Aufzählung der hier möglichen Ausgangsverbindungen IIa. Dies gilt auch für die Ausgangsverbindungen II allgemein, deren wesentliches Kriterium die Anwesenheit einer oder mehrerer olefinischer Doppelbindungen ist, welche bei der Hydrierung der Carbonylgruppe nicht oder nicht nennenswert angegriffen werden sollen. Die sonstige chemische Natur spielt hier keine Rolle, jedoch handelt es sich in der Praxis meist um unverzweigte oder verzweigte ein- oder mehrfach ungesättigte Alkenale und Alkenone mit 4-10 C-Atomen.

## Beispiel 1

Partielle Hydrierung von Citral

Es wurden jeweils 20 g reines Citral unter verschiedenen Bedingungen hydriert, wobei in allen Fällen handelsübliche Trägerkatalysatoren mit Aktivkohle als Träger und 5 Gew.% Metallgehalt verwendet wurden.

Die Reaktionsbedingungen sowie der Verfahrensergebnisse der einzelnen Versuche sind der Tabelle 1 zu entnehmen. Vergleichsversuche wurden hierbei mit « V » gekennzeichnet.

Die Ausbeuten an den Verfahrensprodukten wurden gaschromatographisch, die an Rückstand gravimetrisch bestimmt.

Tabelle 1

| Vers. Nr. | Katalysator Gew.%[3] | Methanol g | Amin g | Druck bar | Temp. °C | Dauer h | Umsatz[1] % | Ausbeute[2] Geraniol/ Nerol | % Citronellol | Rückstand |
|---|---|---|---|---|---|---|---|---|---|---|
| 1 | Ir 0,02 | 16 | $NMe_3$[4] 8 | 100 | 50 | 8 | 100 | 93 | 6 | 1 |
| 1V | Ir 0,02 | 16 | $NMe_3$[4] 8 | 100 | 50 | 5 | 90 | 76 | 23 | 1 |
| 2 | Os 0,02 | 16 | $NMe_3$ 8 | 100 | 50 | 7 | 99 | 92 | 7 | 1 |
| 2V | Os 0,02 | 16 | $NMe_3$ 8 | 100 | 50 | 5 | 100 | 71 | 28 | 1 |
| 3 | Ru 0,02 | 16 | $NMe_3$ 8 | 100 | 50 | 6 | 100 | 93 | 6 | 1 |
| 3V | Ru 0,02 | 16 | $NMe_3$ 8 | 100 | 50 | 5 | 100 | 60 | 39 | 1 |
| 4 | Pt 0,02 | 16 | $NMe_3$ 8 | 100 | 50 | 6 | 100 | 91 | 8 | 1 |
| 4V | Pt 0,02 | 16 | $NMe_3$ 8 | 100 | 50 | 7 | 93 | 71 | 28 | 1 |
| 5 | Rh 0,02 | 16 | $NMe_3$ 8 | 100 | 50 | 7 | 99 | 86 | 12 | 1 |
| 5V | Rh 0,02 | 16 | $NMe_3$ 8 | 100 | 50 | 5 | 100 | 63 | 35 | 2 |
| 6 | Os 0,1 | 16 | $NMe_3$ 8 | 50 | 50 | 8 | 99 | 89 | 10 | 1 |
| 7 | Os 1,1 | 16 | $NMe_3$ 8 | 10 | 50 | 15 | 100 | 93 | 6 | 1 |
| 8 | Os 0,02 | 16 | $NEt_3$[5] 8 | 100 | 100 | 6 | 100 | 92 | 6 | 2 |
| 9 | Os 0,02 | 0 | $NEt_3$[5] 8 | 100 | 100 | 7 | 99 | 91 | 8 | 1 |

1) bezogen auf eingesetzten Aldehyd
2) bezogen auf Umsatz
3) bezogen auf eingesetzten Aldehyd
4) Me = Methyl
5) Et = Ethyl

Tabelle 1

| Vers. Nr. | Katalysator Gew.% | Methanol g | Amin g | | Druck bar | Temp. °C | Dauer h | Umsatz[1] % | Ausbeute Geraniol/ Nerol | % Citronellol | Rückstand |
|---|---|---|---|---|---|---|---|---|---|---|---|
| 10 | Os 0,02 | 16 | $NProp_3$[6] | 8 | 100 | 100 | 5 | 98 | 90 | 9 | 1 |
| 11 | Os 0,02 | 16 | $NBut_3$[7] | 8 | 100 | 100 | 5 | 100 | 90 | 9 | 1 |
| 12 | Os 0,02 | 16 | Pyridin | 8 | 100 | 100 | 6 | 98 | 89 | 9 | 2 |
| 13 | Os 0,02 | 16 | Dimethyl-anilin | 8 | 100 | 100 | 6 | 97 | 89 | 9 | 2 |
| 14 | Os 0,02 | 16 | N-Methyl-Piperidin | 8 | 100 | 100 | 5 | 99 | 92 | 7 | 1 |
| 15 | Ru 0,1 | 16 | $NMe_3$ | 2 | 50 | 100 | 8 | 99 | 88 | 11 | 1 |
| 16 | Ru 0,1 | 16 | $NMe_3$ | 8 | 50 | 100 | 8 | 100 | 93 | 6 | 1 |
| 17 | Ru 0,1 | 16 | $NMe_3$ | 16 | 50 | 100 | 8 | 100 | 95 | 4 | 1 |
| 18 | Ru[8] 0,02 | 16 | $NH_3$ | 8 | 100 | 50 | 6 | 100 | 94 | 5 | 1 |
| 19 | Ru[9] 0,02 | 16 | $NH_3$ | 8 | 100 | 50 | 7 | 100 | 93 | 6 | 1 |
| 20 | Ru[10] 0,02 | 16 | $NH_3$ | 8 | 100 | 50 | 7 | 100 | 94 | 5 | 1 |

6) Prop = n-Propyl
7) But = n-Butyl
8) Katalysator 4 × rückgeführt
9) Katalysator 9 × rückgeführt
10) Katalysator 15 × rückgeführt

Beispiel 2

Partielle Hydrierung von Citronellal

Analog Beispiel 1 wurden jeweils 20 g reines Citronellal (3,7-Dimethyloct-6-en-1-al) hydriert. Die Reaktionsbedingungen sowie die Verfahrensergebnisse sind der Tabelle 2 zu entnehmen.

Tabelle 2

| Vers. Nr. | Katalysator Gew.%[3] | | Methanol g | Amin g | | Druck bar | Temp. °C | Dauer h | Umsatz[1] % | Ausbeute[2] Citronellol | % 3,7-Dimethyl-octanal | Rückstand |
|---|---|---|---|---|---|---|---|---|---|---|---|---|
| 1 | Ir | 0,02 | 16 | $NMe_3$[4] | 8 | 100 | 100 | 4 | 100 | 97 | 1[11] | 1 |
| 2 | Os | 0,02 | 16 | $NMe_3$[4] | 8 | 100 | 100 | 5 | 99 | 97 | 2 | 1 |
| 3 | Pt | 0,02 | 16 | $NMe_3$[4] | 8 | 100 | 100 | 5 | 98 | 96 | 3 | 1 |
| 4 | Ru | 0,02 | 16 | $NMe_3$[4] | 8 | 100 | 100 | 6 | 100 | 95 | 3 | 1 |
| 5 | Ru | 0,02 | 16 | $NMe_3$[4] | 8 | 100 | 100 | 10 | 100 | 98 | 1 | 1 |
| 6 | Rh | 0,02 | 16 | $NMe_3$[4] | 8 | 100 | 100 | 7 | 100 | 92 | 6 | 2 |
| 7 | Ru | 0,02 | 0 | $NEt_3$[5] | 8 | 100 | 100 | 6 | 98 | 97 | 2 | 1 |
| 8 | Ru | 0,02 | 16 | $NEt_3$[5] | 8 | 100 | 100 | 5 | 100 | 97 | 2 | 1 |
| 9 | Ru | 0,02 | 16 | $NBut_3$[7] | 8 | 100 | 100 | 6 | 95 | 98 | 1 | 1 |
| 10 | Ru | 0,02 | 16 | Di-me-Anilin | 8 | 100 | 100 | 7 | 99 | 96 | 3 | 1 |
| 11 | Ru | 0,6 | 16 | $NMe_3$ | 8 | 5 | 50 | 11 | 100 | 97 | 2 | 1 |
| 12 | Ru | 0,01 | 16 | $NMe_3$ | 8 | 100 | 100 | 7 | 100 | 97 | 2 | 1 |
| 13 | Ru[8] | 0,02 | 16 | $NMe_3$ | 8 | 50 | 100 | 9 | 100 | 98 | 1 | 1 |
| 14 | Ru[9] | 0,02 | 16 | $Nme_3$ | 8 | 50 | 100 | 10 | 99 | 97 | 1 | 1 |
| 15 | Ru[10] | 0,02 | 16 | $NMe_3$ | 8 | 50 | 100 | 11 | 100 | 97 | 2 | 1 |

Fußnoten 1) bis 10) s. Tabelle 1
11) bei den Versuchen 1 bis 12 wurden jeweils noch zwischen 0,5 und 1,5 % 2-Isopropenyl-5-methyl-cyclohexen-1-ol (Isophlegol) nachgewiesen.

Beispiele 3-7

Partielle Hydrierung verschiedener α,β-ungesättigter Aldehyde

Analog Beispiel 1 wurden jeweils 20 g verschiedener α,β-ungesättigter Aldehyde zu den entsprechenden ungesättigten Alkoholen hydriert.

Die Versuchsbedingungen und die Verfahrensergebnisse gehen aus Tabelle 3 hervor.

(Siehe die Tabelle 3, Seite 7)

Tabelle 3

| Beispiel Nr. | Aldehyd | Katalysator Gew.%[3)] | | Methanol g | Amin gg | Druck bar | Temp. °C | Dauer h | Umsatz[1)] % | Alkohol | Ausbeute[2)] % | Rück-stand |
|---|---|---|---|---|---|---|---|---|---|---|---|---|
| 3 | Croton-aldehyd | Os | 0,02 | 16 | $NMe_3$[4)] | 7 50 | 50 | 11 | 98 | But-2-en-1-ol | 85 | 5 |
| 4 | 3-Methyl-croton-aldehyd | Ru | 0,02 | 16 | $NMe_3$[4)] | 8 50 | 100 | 12 | 98 | 3-Methylbut-2-en-1ol | 95 | 1 |
| 5 | Zimtaldehyd | Pt | 0,02 | 16 | $NMe_3$[4)] | 8 100 | 100 | 6 | 100 | Zimtalkohol | 96 | 1 |
| 6 | α-Methyl-p-tert.-butylzimt-aldehyd | Ru | 0,02 | 16 | $NMe_3$[4)] | 8 100 | 100 | 8 | 100 | α-Methyl-p-tert.-butyl-zimt-alkohol | 93 | 1 |
| 7 | 3,7-Dime-thyl-nona-2,6-dien-1-al | Ru | 0,02 | 16 | $NMe_3$[4)] | 8 50 | 100 | 9 | 99 | 3,7-Dimethyl-nona-2,6-dien-1-ol | 94 | 1 |

Fußnoten 1) bis 4) s. Tabelle 1

**Ansprüche**

1. Verbessertes Verfahren zur Herstellung von olefinisch ungesättigten Alkoholen (I) durch selektive Hydrierung der entsprechenden ungesättigten Carbonylverbindungen (II) in der Flüssigphase mit Wasserstoff in Gegenwart von Edelmetallkatalysatoren, dadurch gekennzeichnet, daß man

— als Katalysator einen Ruthenium-, Rhodium-, Osmium-, Iridium- oder Platin-Katalysator verwendet und daß man

— die Hydrierung in Gegenwart von 5 bis 40 Gew.%, bezogen auf II, eines tertiären Amins vornimmt.

2. Verfahren nach Anspruch 1, dadurch gekennzeichnet, daß man es auf die Herstellung von ungesättigten Alkoholen der allgemeinen Formel Ia

$$R^1-\overset{R^2}{\overset{|}{C}} = \overset{R^3}{\overset{|}{C}} - \overset{R^4}{\overset{|}{CH}} - OH \qquad \text{(Ia)}$$

in der $R^1$ Wasserstoff oder einen organischen Rest und $R^2$, $R^3$ und $R^4$ Wasserstoff oder eine $C_1$-$C_4$-Alkylgruppe bedeuten, anwendet, indem man von den entsprechenden $\alpha,\beta$-ungesättigten Carbonylverbindungen IIa

$$R^1-\overset{R^2}{\overset{|}{C}} = \overset{R^3}{\overset{|}{C}} - \overset{R^4}{\overset{|}{C}} = O \qquad \text{(IIa)}$$

ausgeht.

3. Verfahren nach Anspruch 2, dadurch gekennzeichnet, daß man es auf die Hydrierung von Citral (IIb) zu Geraniol (E-Ib) und Nerol (Z-Ib) anwendet.

4. Verfahren nach den Ansprüchen 1 bis 3, dadurch gekennzeichnet, daß man als tertiäres Amin Trimethylamin verwendet.

**Claims**

1. An improved process for the preparation of olefinically unsaturated alcohols (I) by selective hydrogenation of the corresponding unsaturated carbonyl compounds (II) in the liquid phase with hydrogen in the presence of a noble metal catalyst, wherein the catalyst used is a ruthenium, rhodium, osmium, iridium or platinum catalyst and the hydrogenation is carried out in the presence of from 5 to 40 % by weight, based on (II), of a tertiary amine.

2. A process as claimed in claim1, wherein an unsaturated alcohol of the general formula Ia

$$R^1-\overset{R^2}{\overset{|}{C}} = \overset{R^3}{\overset{|}{C}} - \overset{R^4}{\overset{|}{CH}} - OH \qquad \text{(Ia)}$$

where $R^1$ is hydrogen or an organic radical and $R^2$, $R^3$ and $R^4$ are hydrogen or $C_1$-$C_4$-alkyl is prepared from the corresponding $\alpha,\beta$-unsaturated carbonyl compounds (IIa)

$$R^1-\overset{R^2}{\overset{|}{C}} = \overset{R^3}{\overset{|}{C}} - \overset{R^4}{\overset{|}{C}} = O \qquad \text{(IIa)}$$

3. A process as claimed in claim 2, wherein citral (IIb) is hydrogenated to geraniol (E-Ib) and nerol (Z-Ib).

4. A process as claimed in claims 1 to 3, wherein trimethylamine is used as the tertiary amine.

**Revendications**

1. Procédé perfectionné pour préparer des alcools à insaturation oléfinique I par hydrogénation sélective des composés carbonylés insaturés correspondants II en phase liquide, à l'aide d'hydrogène en présence de catalyseurs à base de métaux nobles, caractérisé en ce qu'on utilise en tant que catalyseur un catalyseur au ruthénium, au rhodium, à l'osmium, à l'iridium ou au platine, et on effectue l'hydrogénation en présence de 5 à 40 % en poids, par rapport au composé II, d'une amine tertiaire.

2. Procédé selon la revendication 1, caractérisé en ce qu'on l'applique à la préparation des alcools insaturés de formule générale Ia

$$R^1-\overset{R^2}{\overset{|}{C}} = \overset{R^3}{\overset{|}{C}} - \overset{R^4}{\overset{|}{CH}} - OH \qquad \text{(Ia)}$$

dans laquelle $R^1$ représente l'hydrogène ou un reste organique et $R^2$, $R^3$ et $R^4$ représentent l'hydrogène ou un groupe alkyle en $C_1$-$C_4$, en partant des composés carbonylés alpha, bêta-insaturés correspondants IIa

$$R^1-\overset{R^2}{\overset{|}{C}}=\overset{R^3}{\overset{|}{C}}-\overset{R^4}{\overset{|}{C}}=O \qquad \text{(IIa)}$$

3. Procédé selon la revendication 2, caractérisé en ce qu'on l'applique à l'hydrogénation du citral IIb en géraniol E-Ib et nérol Z-Ib.

4. Procédé selon l'une quelconque des revendications 1 à 3, caractérisé en ce que l'on utilise en tant qu'amine tertiaire la triméthylamine.